# FASCICULE DE BREVET EUROPEEN

(11) **EP 2 223 680 B1**
(45) Date de publication et mention de la délivrance du brevet: **18.10.2017**
(21) Numéro de dépôt: 09181015.0
(22) Date de dépôt: 30.12.2009
(51) Int. Cl.: A61K 8/365, A61K 8/368, A61K 8/60, A61Q 19/00, A61Q 19/08, A61K 8/36

(54) **Association de monosaccharides et d'agents desquamants et son utilisation en cosmétique**
Kombination aus Monosaccharide und ein Mittel zum entfernen von Hautschuppen und ihre kosmetische Verwendung
Combination of monosaccharides and exfoliating agents and its cosmetic use

(30) Priorité: 30.12.2008 FR 0859149; 15.01.2009 US 144755 P
(43) Date de publication de la demande: 01.09.2010
(73) Titulaire: L'Oréal, 75008 Paris (FR)
(72) Inventeur: Laboureau, Julien, 92130, Issy les Moulineaux (FR); Simonnet, Jean-Thierry, 94230, Cachan (FR); Portes, Pascal, 94130, Nogent sur Marne (FR)
(74) Mandataire: Tezier Herman, Béatrice

(56) Documents cités:
- EP-A1- 0 756 866
- EP-A2- 0 378 936
- EP-A2- 0 704 210
- EP-A2- 1 333 022
- WO-A1-2007/101493
- WO-A1-2008/068297
- FR-A1- 2 739 556
- FR-A1- 2 767 694
- FR-A1- 2 768 623
- FR-A1- 2 853 539
- FR-A1- 2 876 283
- FR-A1- 2 900 574
- US-A1- 2007 025 933
- DATABASE GNPD [Online] MINTEL; juillet 2006 (2006-07), "Exfoliating cream", XP002692267, Database accession no. 557651
- DATABASE GNPD MINTEL; mars 2008 (2008-03), "Lightening serum", XP002692268, Database accession no. 882731
- DATABASE GNPD [Online] MINTEL; octobre 2006 (2006-10), "Rich cream", XP002692269, Database accession no. 597642

## Description

La présente invention concerne l'utilisation d' une composition, notamment cosmétique et/ou dermatologique, comprenant dans un milieu physiologiquement acceptable, l'association d'au moins un monosaccharide choisi parmi le mannose, le rhamnose et leur mélange, et d'au moins un agent desquamant.

La peau humaine est constituée de deux couches principales qui sont le derme et l'épiderme qui recouvre le derme de manière superficielle. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac...), appelé « fonction barrière ».

L'épiderme est un épithélium pavimenteux stratifié kératinisé constitué à 90% de kératinocytes. La différenciation progressive des cellules de la membrane basale, qui sépare le derme de l'épiderme, vers la surface de l'épiderme comprend notamment la différenciation des kératinocytes qui migrent vers la surface de la peau où ils desquament.

Le vieillissement de l'épiderme se manifeste principalement par la réduction de son épaisseur. L'atrophie de l'épiderme est la conséquence du ralentissement de la prolifération des kératinocytes et de l'accumulation de kératinocytes sénescents. La couche cornée devient terne. Le phénomène de desquamation s'altère ce qui participe à la diminution de la régénération épidermique. Ainsi, une élimination forcée de la couche cornée accélère le renouvellement et permet de lutter contre le vieillissement.

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. La cohésion entre l'épiderme et le derme est assurée par la jonction dermo-épidermique. C'est une région complexe d'une épaisseur de 100 nm environ qui comprend le pole basal des kératinocytes basaux, la membrane épidermique et la zone sous-basale du derme superficiel.

Les collagènes sont les protéines majoritaires des matrices extracellulaires de la peau. A ce jour, 20 types de collagènes sont identifiés et notés de I à XX. Les collagènes majoritairement présents dans l'ensemble du derme sont les collagènes de type I et III qui forment la matrice extracellulaire de l'ensemble du derme (ce sont ces collagènes qui constituent 70-80 % du poids sec du derme). Par ailleurs, les collagènes ne sont pas tous synthétisés par les mêmes types cellulaires, les collagènes de type I et III sont essentiellement produits par les fibroblastes dermiques alors que le collagène de type VII est produit par deux catégories de cellules, les kératinocytes et les fibroblastes. La régulation de leur expression diffère d'un collagène à un autre, par exemple, les collagènes I et VII ne sont pas régulés de la même manière par certaines cytokines, en effet, le TNF alpha et la leukoréguline stimulent le collagène VII et régulent négativement le collagène I. Enfin, toutes les molécules de collagène sont des variantes d'un précurseur commun qui est la chaîne alpha du procollagène.

Avec le vieillissement, le collagène s'amincit et des rides apparaissent à la surface de la peau. Le vieillissement cutané est un mécanisme génétiquement programmé.

Par ailleurs, certains facteurs d'environnement comme le tabagisme et surtout l'exposition au rayonnement du soleil l'accélèrent. La peau a ainsi un aspect beaucoup plus âgé sur les zones exposées au soleil comme le dos des mains ou le visage. Ainsi, ces autres facteurs ont également un impact négatif sur le collagène naturel de la peau.

En conséquence, compte tenu du rôle important du collagène au niveau de l'intégrité de la peau et de sa résistance aux agressions externes de type mécaniques, la stimulation de synthèse de ces collagènes, et en particulier du collagène de type I, apparaît comme un moyen efficace pour pallier les signes du vieillissement cutané. Au cours du vieillissement chronologique et/ou actinique, l'épiderme subit également de nombreuses modifications et dégradations qui se traduisent, avec l'âge, par une altération du microrelief, une apparition de rides et ridules, une altération des propriétés mécaniques de la peau, notamment un manque d'élasticité de la peau, et une perte d'éclat du teint.

On comprend donc l'importance de pouvoir disposer de produits dont les effets visent à régénérer le tissu de la peau via une augmentation de la prolifération des kératinocytes, une stimulation de la prolifération et/ou du métabolisme des fibroblastes, et notamment une stimulation de la synthèse des collagènes.

A cet égard, la demanderesse a trouvé que le mannose ou le rhamnose permet l'activation de la prolifération des kératinocytes et/ou des fibroblastes et/ou stimule la synthèse du procollagène I. L'utilisation de compositions les contenant permet ainsi de contrer les signes du vieillissement cutané, et en particulier l'atrophie épidermique et/ou dermique liée au vieillissement. En y associant des agents favorisant la desquamation, c'est-à-dire l'élimination des cellules mortes situées à la surface de la couche cornée de l'épiderme, cela permet au mannose ou au rhamnose de favoriser la pénétration des monosaccharides de l'invention, et donc optimiser leur efficacité biologique, à savoir de lutter contre l'apparition des signes du chrono et photovieillissement.

L'action conjointe des monosaccharides sélectionnés avec au moins un agent desquamant permet de stimuler doublement le renouvellement épidermique, en agissant pour les monosaccharides selon l'invention, sur la prolifération des kératinocytes malpighiens (épiderme vivant) et sur la desquamation des cornéocytes (Stratum Corneum) pour les agents desquamants.

L'utilisation de ces monosaccharides était restée jusqu'alors inconnue pour les effets biologiques directs exposés plus haut. La demande WO 2007/128939 mentionne toutefois une activité anti-âge obtenue par un effet biomécanique d'un agent tenseur en association avec des composés saccharidiques, qui permettent d'augmenter l'expression des mécanorécepteurs de cellules de la peau. Cette augmentation de l'expression des mécanorécepteurs est décrite comme augmentant la sensibilisation des cellules de la peau à répondre aux effets des tenseurs.

La demande de brevet US 2007/0025933 décrit une composition comprenant une base photoprotectrice, constituée de deux types de composants, et éventuellement un mélange de composants additionnels, tels que notamment des monosaccharides (comme le mannose, fructose et glucose) et des acides du cycle de Krebs ou leurs dérivés (comme l'acde citrique, malique, fumarique) pour stabiliser ladite composition. Aucune activité propre aux monosaccharides sur la peau n'est mentionnée.

La demande WO 2005/063194 décrit une base galénique à très haute tolérance comprenant notamment du mannose ou du rhamnose. Il est spécifié qu'une telle base galénique ne peut fonctionner qu'en association avec un actif dont elle est seulement le véhicule. Les bases galéniques dermiques et/ou cosmétiques divulguées reposent essentiellement sur la présence des deux polyols que sont le mannitol et le xylitol.

La présente invention concerne donc l'utilisation d' une composition, notamment cosmétique et/ou dermatologique, comprenant l'association d'au moins un monosaccharide choisi parmi le mannose, le rhamnose et leur mélange avec au moins un agent desquamant pour diminuer et/ou prévenir les caractéristiques des rides et/ ou ridules ou pour améliorer la densité de la peau et/ou sa fermeté. Le mannose est un hexose épimère en C2 du glucose. Le rhamnose (ou 6 deoxy mannose) constitue formellement le produit de désoxygénation du mannose en C6. Les monosaccharides selon l'invention sont sous la forme D ou L du mannose et/ou du rhamnose ou leur mélange, chaque forme pouvant elle-même être l'anomère alpha et/ou béta. Les formes préférées selon l'invention sont le D-mannose ou le L-rhamnose.

Le D-mannose est présent dans les végétaux en particulier certains fruits dont les airelles (canneberges), ou le bois dur (hêtre, bouleau). Le rhamnose est trouvé dans la nature sous forme L. Le D-mannose, ainsi que le L-rhamnose, sont commercialisés, par exemple, par la société Danisco Sweeteners® et Symrise. Dans la présente invention, le monosaccharide est sous forme de monomère. L'agent desquamant dans la présente invention est choisi parmi les dérivés de l'acide salicylique, l'acide jasmonique ou ses dérivés, et l'acide gentisique ou ses dérivés. Les dérivés d'acides salicylique sont choisis parmi l'acide n-octanoyl-5-salicylique (ou acide capryloyl salicylique) ; l'acide n-décanoyl -5- salicylique ; l'acide n-dodécanoyl-5-salicylique ; l'acide n-heptyloxy-5-salicylique et leurs sels correspondants.

Le composé d'acide salicylique est avantageusement choisi parmi l'acide salicylique et l'acide n-octanoyl-5-salicylique. On utilisera plus particulièrement l'acide n-octanoyl-5- salicylique. Les dérivés d'acide salicylique tels que décrits précédemment peuvent être présents dans la composition selon l'invention en une teneur allant de 0,05 % à 10 % en poids, par rapport au poids total de la composition, de préférence allant de 0,05 % à 5 % en poids, et préférentiellement allant de 1 % à 4 % en poids.

De manière particulière, l'agent desquamant est choisi parmi ceux décrits dans les demandes de brevets FR2581542, WO 98/35973, FR2759370, FR2762839 ou EP0875495.

Selon un autre mode de l'invention, l'agent desquamant présent dans la composition selon l'invention est choisi parmi l'acide jasmonique et ses dérivés de formule (II) suivante : 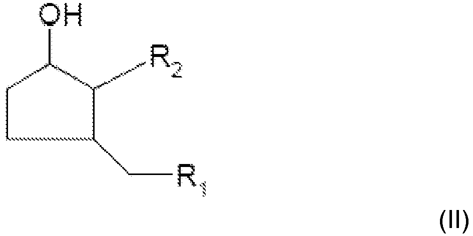 dans laquelle :
- R1 est un radical choisi parmi -COOR', -CONR'R", -CH2OR', -COR', -CH2R', - SO2OR', -PO3R'R", -NHR' avec R' et R", indépendamment l'un de l'autre, désignant un atome d'hydrogène ou un radical hydrocarboné, linéaire, ramifié ou cyclique, saturé ou insaturé, ayant 1 à 18 atomes de carbone, éventuellement substitué par 1 à 5 groupements, identiques ou différents, choisis parmi -OR"',-OCOR''', -SR''', -SCOR''', NR"'R"", -NHCOR''', -Halogène, -CN, -COOR''', -COR"' avec R''' et
   R"" représentant, indépendamment l'un de l'autre, un atome d'hydrogène, un radical
   aryle ou un radical hydrocarboné, linéaire ou ramifié, saturé ou insaturé ayant 1 à 4 atomes de carbone;
- R2 est un radical hydrocarboné, linéaire, ramifié ou cyclique, saturé ou insaturé, ayant 1 à 18 atomes de carbone, éventuellement substitué par 1 à 5 groupements, identiques ou différents, choisis parmi -OR"', -OCOR''', -SR''', -SCOR''', NR"'R"",-NHCOR"', -Halogène, -CN, -COOR''', -COR''' avec R"' et R"" représentant, indépendamment l'un de l'autre, un atome d'hydrogène, un radical aryle ou un radical hydrocarboné, linéaire ou ramifié, saturé ou insaturé ayant 1 à 4 atomes de carbone; choisi parmi:
   - l'acide jasmonique,
   - l'acide 3-hydroxy-2-[(2Z)-2-pentenyl]- cyclopentaneacétique,
   - le 3-hydroxy-2-[(2Z)-2-pentenyl]- cyclopentaneacétate de méthyle,
   - le 2-[(2Z)-2-pentenyl]-3-hydroxy-cyclopentaneéthanol,
   - l'acide 3-hydroxy-2-pentyl-cyclopentaneacétique,
   - le 3-hydroxy-2-pentyl-cyclopentaneacétate de méthyle, et
   - le 2-pentyl-3-hydroxy-cyclopentaneéthanol.

La quantité de composé de formule (II) utilisable selon l'invention dépend bien évidemment de l'effet recherché et doit être en une quantité efficace pour favoriser la desquamation de la peau et/ou stimuler le renouvellement épidermique et donc lutter contre le vieillissement cutané intrinsèque et/ou extrinsèque.

A titre d'exemple, la quantité de composé de formule (II) utilisable selon l'invention peut aller par exemple de 0,01 à 30% et de préférence de 0,5 à 15%, et notamment de 1 à 5% en poids, par rapport au poids total de la composition.

On peut également se référer aux dérivés de l'acide jasmonique tels que définis dans les demandes de brevets FR2835526, EP 1333022, EP 1333021, EP 1442737, EP 1502909, FR2835525, FR2858320, ou FR2850571.
Parmi les dérivés de l'acide salicylique, on peut également se référer aux composés tels que définis dans les demandes de brevets FR2737410 ou EP 0756866.

Lorsque le composé additionnel présent dans la composition selon l'invention est l'acide salicylique, l'acide citrique ou un de ses sels, comme le citrate trisodique, la composition selon l'invention est en particulier dépourvue de xylitol et de mannitol. Selon une autre alternative, le composé additionnel présent dans la composition selon l'invention n'est pas l'acide salicylique, l'acide citrique ou un de ses sels.

Selon un mode particulier de réalisation de l'invention, la composition selon l'invention ne comprend pas l'association de xylitol et de mannitol.

La présente invention porte aussi sur l'utilisation, notamment cosmétique ou dermatologique, d'une composition selon l'invention, administrée par voie orale, topique ou par injection cutanée, notamment pour le soin de la peau et/ou du cuir chevelu.

Une composition conforme à l'invention telle que définie précédemment peut notamment être une composition cosmétique pour soin capillaire pour, en particulier, la stimulation de la pousse du cheveu, la lutte contre la chute des cheveux, le ralentissement de sa chute ou le renforcement de l'éclat du cheveu.

La présente invention a également pour objet une méthode de traitement, en particulier cosmétique ou thérapeutique, pour diminuer ou prévenir les signes du vieillissement de la peau ou de ses phanères (cheveux, cils, ongles, ...), par administration à un sujet, de préférence un être humain, d'une quantité efficace d'au moins un monosaccharide tel que défini précédemment en association avec une quantité efficace d'au moins un composé additionnel tel que défini précédemment. L'invention a en particulier pour objet un procédé de traitement cosmétique d'une peau ridée, en particulier la peau du visage et/ou du front, comprenant l'application topique sur ladite peau d'une composition comprenant, dans un milieu physiologiquement acceptable, l'association d'une quantité efficace d'au moins un monosaccharide tel que défini précédemment et d'une quantité efficace d'au moins un agent desquamant.

La présente invention concerne également l'utilisation, notamment cosmétique ou dermatologique, de la composition ou de l'assocation selon l'invention, pour diminuer et/ou prévenir les signes du vieillissement de la peau ou de ses phanères.

L'invention a en particulier pour objet l'utilisation cosmétique d'une composition comprenant, dans un milieu physiologiquement acceptable, l'association d'au moins un monosaccharide choisi parmi le mannose, le rhamnose et leur mélange, et d'au moins un agent desquamant, pour améliorer l'éclat du teint, pour diminuer et/ou prévenir les caractéristiques des rides et/ou ridules, pour améliorer et/ou diminuer le micro-relief de la peau, et/ou pour lisser la peau et/ou pour favoriser la desquamation de la peau et/ou stimuler le renouvellement épidermique. Plus spécifiquement, elle concerne l'utilisation cosmétique de ladite association pour diminuer et/ou prévenir les caractéristiques des rides et/ou ridules, pour améliorer et/ou diminuer le micro-relief de la peau, et/ou pour lisser la peau et/ou pour favoriser la desquamation de la peau et/ou stimuler le renouvellement épidermique.

Selon un mode particulier, la composition utilisée dans la cadre de la présente invention ne comprend pas l'association de xylitol et de mannitol.

La composition ou l'association selon l'invention permet aussi de stimuler la régénération des cellules de l'épiderme et du derme, au niveau de la peau ou des phanères, en particulier les kératinocytes et les fibroblastes, notamment par augmentation de leur prolifération. On dispose de la sorte d'une méthode, notamment cosmétique, efficace notamment pour lutter contre les signes du chronovieillissement.

Les signes du chronovieillissement correspondent aux dégradations internes de la peau dues au vieillissement intrinsèque des individus.

Selon une forme de réalisation préférée, l'utilisation selon la présente invention est destinée à améliorer l'éclat du teint, à diminuer et/ou prévenir les caractéristiques des rides et/ou ridules, à améliorer et/ou diminuer le micro-relief de la peau, et/ou à lisser la peau et/ou à favoriser la desquamation de la peau et/ou à stimuler le renouvellement épidermique, notamment en agissant sur la prolifération des kératinocytes et sur la desquamation de la peau, et en particulier des cornéocytes.

Selon un autre aspect de l'invention, l'utilisation de la composition ou de l'association selon l'invention permet d'améliorer la densité de la peau et/ou sa fermeté.

La présente invention concerne également l'utilisation de la composition ou de l'association selon l'invention pour traiter, de manière préventive ou curative, les rides et/ou ridules, la peau flétrie, le manque d'élasticité et/ou de tonus de la peau, l'amincissement du derme, la dégradation des fibres de collagène, la peau molle et/ou la peau amincie.

La quantité des ingrédients actifs, choisis parmi les monosaccharides et les agents desquamants précédemment définis, à mettre en oeuvre selon l'invention dépend de l'effet cosmétique ou thérapeutique recherché, et peut donc varier dans une large mesure. L'homme de l'art peut aisément, sur la base de ses connaissances générales, déterminer les quantités appropriées.

Ainsi, et selon une forme de réalisation préférée, la composition selon l'invention comprend au moins un monosaccharide tel que défini ci-dessus en une quantité comprise entre 0,001 % et 30 % en poids par rapport au poids total de la composition, et en particulier entre 0,1% et 10 % en poids, et plus particulièrement entre 0,5 % et 6 % en poids par rapport au poids total de la composition.

Selon une forme de réalisation préférée, la composition selon l'invention comprend un agent desquamant en une quantité comprise entre 0,001 % et 30 % en poids par rapport au poids total de la composition, et en particulier entre 0,1 % et 10 % en poids, et plus particulièrement entre 0,5 % et 6 % en poids par rapport au poids total de la composition.

La composition selon l'invention est adaptée à une administration topique sur la peau ou ses phanères, une administration orale ou une injection cutanée, en particulier sous la forme d'une solution stérile.

De préférence, les administrations topiques selon l'invention se présentent sous forme d'une crème, d'un gel, d'une lotion, d'un lait, d'une huile, d'un onguent, d'une cire, d'une mousse, d'une pâte, d'un sérum, d'une pommade ou d'un shampooing.

De manière également préférée, les administrations orales selon l'invention se présentent sous la forme d'une gélule, d'un comprimé, ou de pilules.

Le monosaccharide selon l'invention, et l'agent desquamant, sont plus particulièrement présents dans la composition selon l'invention à titre d'agent (ou ingrédient) actif, en particulier de seuls agents actifs.

Par « agent actif » ou « ingrédient actif », on entend plus spécifiquement selon l'invention, un composé qui, lorsqu'il est administré à un sujet, en particulier un sujet humain, joue un rôle biologique direct sur l'organisme, en particulier sur la peau ou ses phanères, en particulier sans améliorer l'effet biologique ou mécanique d'un autre composé présent dans la composition selon l'invention.

D'une manière générale, le milieu dans lequel sont compris les principes actifs de la composition tel que définie précédemment, est un milieu physiologiquement acceptable, en particulier un milieu cosmétiquement ou pharmaceutiquement acceptable et peut être anhydre ou aqueux. Il peut ainsi comprendre une phase aqueuse et/ou une phase grasse.

Le milieu physiologiquement acceptable dans lequel les composés selon l'invention peuvent être employés, ainsi que ses constituants, leur quantité, la forme galénique de la composition, son mode de préparation, et son mode d'administration peuvent être choisis par l'homme du métier sur la base de ses connaissances générales en fonction du type de composition recherchée.

Lorsque la composition est une composition destinée à une administration topique, elle peut se présenter avantageusement sous la forme de solutions aqueuses, hydroalcooliques, d'émulsions huile-dans-eau (H/E) ou eau-dans-huile (E/H) ou multiple (triple : E/H/E ou H/E/H), des nanoémulsions, en particulier des nanoémulsions H/E, dont la tailles des gouttes est inférieure à 100nm, de gels aqueux, ou de dispersions d'une phase grasse dans une phase aqueuse à l'aide de sphérules, ces sphérules pouvant être des nanoparticules polymériques telles que les nanosphères et les nanocapsules ou des vésicules lipidiques de type ionique et/ou non ionique (liposomes, niosomes, oléosomes).

Ces compositions sont préparées selon les méthodes usuelles.

En outre, les compositions utilisables selon l'invention peuvent être plus ou moins fluides et avoir l'aspect d'une crème blanche ou colorée, d'une pommade, d'un lait, d'une lotion, d'un sérum, d'une pâte ou d'une mousse. Elles peuvent être éventuellement appliquées sur la peau sous forme d'aérosol. Elles peuvent aussi se présenter sous forme solide, et par exemple sous forme de stick.

Pour une application locale sur les cheveux ou le cuir chevelu, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Lorsque la composition est une émulsion, la proportion de la phase grasse peut varier d'environ 5 % à 80 % en poids, et de préférence d'environ 2 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

La phase huileuse peut comprendre aussi tout additif habituel liposoluble ou lipodispersible comme indiqué ci-après.

Elle peut notamment comprendre des corps gras tels que des cires, des composés pâteux, des alcools gras, des acides gras. La phase huileuse contient au moins une huile, plus particulièrement au moins une huile cosmétique. On entend par « huile » un corps gras liquide à la température ambiante (25°C).

Comme huiles utilisables dans la composition de l'invention, on peut citer par exemple :
- les huiles hydrocarbonées d'origine animale, telles que le perhydrosqualène;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité, le caprylyl glycol ;
- les esters et les éthers de synthèse, notamment d'acides gras, comme les huiles de formules R1 COOR2 et R1OR2 dans laquelle R1 représente le reste d'un acide gras ou d'unalcool gras comportant de 8 à 29 atomes de carbone, et R2 représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle, l'isopropyl lauroyl sarcosinate, notamment vendu sous le nom commercial Eldew SL 205 de la société Ajinomoto ;
- les hydrocarbures linéaires ou ramifiés, d'origine minérale ou synthétique, tels que les huiles de paraffine, volatiles ou non, et leurs dérivés, la vaseline, les polydécènes, l'isohexadécane, l'isododécane, le polyisobutène hydrogéné tel que l'huile de Parléam, le mélange de n-undécane (C11) et de n-tridécane (C13) commercialisé sous la référence de CETIOL UT par la Société Cognis ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; lespolydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; - leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldiméthicone et la trifluoropropyldiméthicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les diméthicone copolyols tels que le mélange de cyclométhicone et de diméthicone copolyol, vendu sous la dénomination « DC 5225 C » par la société Dow Corning, et les alkyl-diméthicone copolyols tels que le Laurylméthicone copolyol vendu sous la dénomination « Dow Corning 5200 Formulation Aid » par la société Dow Corning et le cétyl diméthicone copolyol vendu sous la dénomination « Abil EM 90® » par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

Ces compositions peuvent être également des émulsions H/E stabilisées par des particules comme par exemple des particules polymériques décrites dans le brevet FR2760641, des polymères amphiphiles réticulés ou non tels que décrits dans les demandes : FR2853543 et FR2819175.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Comme solvants utilisables dans l'invention, on peut citer les alcools inférieurs notamment l'éthanol, l'isopropanol, le dipropylène glycol, le butylène glycol et le propylène glycol.

Comme gélifiants hydrophiles utilisables dans l'invention, on peut citer à titre d'exemples non limitatifs, les polymères carboxyvinyliques (carbomer^{®}), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

Lorsque la composition est administrée par voie orale, elle est avantageusement sous la forme d'une gélule, d'un comprimé, ou de pilules. Lorsque la composition est administrée par injection cutanée, elle est en particulier sous la forme d'une solution stérile.

Les compositions de l'invention peuvent contenir d'autres actifs hydrophiles ou lipophiles. Ces actifs sont notamment choisis parmi les agents anti-oxydants, les agents dermo-relaxants ou dermodécontractants, les agents anti-âge, les agents anti-glycation, les agents stimulants la synthèse de macromolécules dermiques ou épidermiques et/ou empêchant leur dégradation, les agents stimulants la prolifération des fibroblastes ou des kératinocytes et/ou la différenciation des kératinocytes, les agents favorisant la maturation de l'enveloppe cornée, les inhibiteurs de NO synthase et les agents stimulant le métabolisme énergétique des cellules. Des listes de ces actifs sont données à la suite à titre illustratif, et ne doivent en aucune façon être considérées comme limitatives.

### Agents anti-âge :

Parmi les actifs connus pour lutter contre les signes du vieillissement, notamment cutané, on peut citer notamment :
   la vitamine B3, le coenzyme Q10 (ou ubiquinone), la vitamine B9, la vitamine E, les dérivés de la vitamine E tels que le dérivé phosphaté comme par exemple le TPNA^{®} commercialisé par la société Showa Denko, le resvératrol ou ses dérivés, comme par exemple le resveratrate^{®} commercialisé par la société Estée Lauder, le rétinol ou ses dérivés, et leur mélange.

### Agents anti-glycation:

Par « agent anti-glycation », on entend un composé prévenant et/ou diminuant la glycation des protéines de la peau, en particulier des protéines du derme, telles que le collagène.

Comme agents anti-glycation, on peut citer notamment les extraits végétaux de la famille des *Ericaceae,* tels qu'un extrait de myrtille (*Vaccinium angusfifollium, Vaccinium myrtillus*), par exemple celui vendu sous la dénomination « BLUEBERRY HERBASOL EXTRACT PG » par la société COSMETOCHEM, l'ergothionéine et ses dérivés, les hydroxystilbènes et leurs dérivés, tels que le resvératrol et le 3,3', 5,5'-tétrahydroxystilbène (ces agents anti-glycation sont décrits dans les demandes FR 2 802 425, FR 2 810 548, FR 2 796 278 et FR 2 802 420, respectivement), les dihydroxystilbènes et leurs dérivés, les polypeptides d'arginine et de lysine tels que celui vendu sous la dénomination « AMADORINE^{®} » par la société SOLABIA, le chorhydrate de carcinine (commercialisé par Exsymol sous la dénomination « ALISTIN^{®}»), un extrait d'*Hélianthus annuus* comme l'Antiglyskin^{®} de SILAB, les extraits de vin tel que l'extrait de vin blanc en poudre sur support maltodextrine vendu sous la dénomination « Vin blanc déshydraté 2F » par la société Givaudan, l'acide thioctique (ou acide alpha lipoïque), un mélange d'extrait de busserole et de glycogène marin comme l'Aglycal LS 8777^{®} de Laboratoires Sériobiologiques, un extrait de thé noir comme le Kombuchka^{®} de Sederma et leurs mélanges.

Comme agents anti-glycation préférés, on citera les extraits de myrtille (*Vaccinium myrtillus*) et l'extrait de thé noir.

### Agents stimulant la synthèse de macromolécules dermiques et/ou épidermiques et/ou empêchant leur dégradation :

Parmi les actifs stimulant les macromolécules du derme ou empêchant leur dégradation, on peut citer ceux qui agissent :
- soit sur la synthèse du collagène, tels que les extraits de Centella asiatica, les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine^{®} ; les peptides de riz tel que le Nutripeptide^{®} de SILAB, le méthylsilanol mannuronate tel que l'Algisium C^{®} commercialisé par Exsymol ; les hormones végétales telles que les auxines et les lignanes ; l'acide folique ; et un extrait de Medicago sativa (alfafa) tel que celui commercialisé par SILAB sous la dénomination Vitanol^{®}; un extrait peptidique de noisette tel que celui commercialisé par la société Solabia sous la dénomination Nuteline C^{®}; et l'arginine.
- soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol^{®} de Silab, un extrait d'aphanizomenon flos-aquae (cyanophycée) commercialisée sous la dénomination Lanablue^{®} par Atrium Biotechnologies, les oligopeptides et les lipopeptides, les lipoaminoacides, l'extrait de malt commercialisé par la société COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB^{®}), de trèfle rouge, de lin, de kakkon; un extrait de litchi ; la DIPALMITOYL HYDROXYPROLINE commercialisé par Seppic sous le nom SEPILIFT DPHP^{®} : Baccharis genistelloide ou Baccharine commercialisé par SILAB, un extrait de moringa tel que l'Arganyl LS 9781^{®} de Cognis ; l'extrait de sauge décrit dans la demande FR-A-2812544 de la famille des labiées (*salvia officinalis* de la société Flacksmann), l'extrait de Rhododendron, le blueberry extract, un extrait de *vaccinium myrtillus* tel que ceux décrits dans la demande FR-A-2814950.
- soit sur la synthèse de molécules appartenant à la famille des élastines (élastine et fibrilline), tels que : le rétinol et dérivés en particulier le palmitate de rétinol ; l'extrait de *Saccharomyces Cerivisiae* commercialisé par la société LSN sous la dénomination commerciale Cytovitin^{®} ; et l'extrait d'algue *Macrocystis pyrifera* commercialisé par la société SECMA sous la dénomination commerciale Kelpadelie^{®}; un extrait peptidique de noisette tel que celui commercialisé par la société Solabia sous la dénomination Nuteline C^{®}.
- soit sur l'inhibition de la dégradation de l'élastine tels que l'extrait peptidique de graines de *Pisum sativum* commercialisé par la société LSN sous la dénomination commerciale Parelastyl^{®} ; les héparinoïdes ; et les composés N-acylaminoamides décrits dans la demande WO 01/94381 tels que l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acétyl-N-[3-(trifluorométhyl)phényl]valyl-glycine ou acétyl trifluorométhyl phényl valylglycine, ou un ester de celui-ci avec un alcool en C₁-C₆. ; un extrait de peptides de riz tel que la Colhibin^{®} de Pentapharm, ou un extrait de Phyllanthus emblica tel que l'Emblica^{®} de Rona.
- soit sur la synthèse des glycosaminoglycannes, tels que le produit de fermentation du lait par lactobacillus vulgaris, commercialisé par la société BROOKS sous la dénomination commerciale Biomin yogourth^{®} ; l'extrait d'algue brune Padina pavonica commercialisé par la société ALBAN MÜLLER sous la dénomination commerciale HSP3^{®} ; l'extrait de *Saccharomyces cerevisiae* disponible notamment auprès de la société SILAB sous la dénomination commerciale Firmalift^{®} ou auprès de la société LSN sous la dénomination commerciale Cytovitin^{®.} ; un extrait de *Laminaria ochroleuca* tel que la Laminaïne^{®} de Secma ; l'essence de Mamaku de Lucas Meyer, un extrait de cresson (Odraline^{®} de Silab).
- soit sur la synthèse de la fibronectine, tels que l'extrait de zooplancton Salina commercialisé par la société SEPORGA sous la dénomination commerciale GP4G^{®} , l'extrait de levure disponible notamment auprès de la société ALBAN MÜLLER sous la dénomination commerciale Drieline^{®} , et le palmitoyl pentapeptide commercialisé par la société SEDERMA sous la dénomination commerciale Matrixil^{®}.

Parmi les actifs stimulant les macromolécules épidermiques, telles que la fillagrine et les kératines, on peut citer notamment l'extrait de lupin commercialisé par la société SILAB sous la dénomination commerciale Structurine^{®} , l'extrait de bourgeons de hêtre *Fagus sylvatica* commercialisé par la société GATTEFOSSE sous la dénomination commerciale Gatuline^{®} RC; et l'extrait de zooplancton Salina commercialisé par la société SEPORGA sous la dénomination commerciale GP4G^{®}; Tripeptide de Cuivre de PROCYTE ; un extrait peptidique de *Voandzeia substerranea* tel que celui commercialisé par la société Laboratoires Sérobiologiques sous la dénomination commerciale Filladyn LS 9397^{®}.

De préférence, on utilisera un actif stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un actif stimulant la synthèse des glycosaminoglycanes choisi parmi un extrait d'algue brune Padina pavonica, un extrait de *Saccharomyces cerevisiae,* un extrait de *Laminaria ochroleuca,* l'essence de Mamaku, un extrait de cresson et leurs mélanges.

Comme actifs préférés stimulant la synthèse de macromolécules dermiques et/ou épidermiques et/ou empêchant leur dégradation, on peut citer :
les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine^{®} ; les peptides de riz tel que le Nutripeptide^{®} de SILAB, le méthylsilanol mannuronate tel que l'Algisium C^{®} commercialisé par Exsymol ; l'acide folique ; un extrait de Medicago sativa (alfafa) tel que celui commercialisé par SILAB sous la dénomination Vitanol^{®}; un extrait peptidique de noisette tel que celui commercialisé par la société Solabia sous la dénomination Nuteline C^{®}; l'arginine ; un extrait d'aphanizomenon flos-aquae (cyanophycée) commercialisée sous la dénomination Lanablue^{®} par Atrium Biotechnologies, l'extrait de malt commercialisé par la société COLETICA sous la dénomination commerciale Collalift^{®} le lycopène ; un extrait de litchi ; un extrait de moringa tel que l'Arganyl LS 9781^{®} de Cognis ; un extrait de *vaccinium myrtillus* tel que ceux décrits dans la demande FR-A-2814950 ; le retinol et dérivés en particulier le palmitate de rétinol ; l'extrait de *Saccharomyces Cerivisiae* commercialisé par la société LSN sous la dénomination commerciale Cytovitin^{®} ; un extrait peptidique de noisette tel que celui commercialisé par la société Solabia sous la dénomination Nuteline C^{®}; l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valylglycine ou acetyl trifluoromethyl phenyl valylglycine, ou un ester de celui-ci avec un alcool en C₁-C_{6.} ; un extrait de peptides de riz tel que la Colhibin^{®} de Pentapharm, ou un extrait de Phyllanthus emblica tel que l'Emblica^{®} de Rona ; l'extrait d'algue brune Padina pavonica commercialisé par la société ALBAN MÜLLER sous la dénomination commerciale HSP3^{®} ; l'extrait de *Saccharomyces cerevisiae* disponible notamment auprès de la société SILAB sous la dénomination commerciale Firmalift^{®} ou auprès de la société LSN sous la dénomination commerciale Cytovitin^{®} ; un extrait de *Laminaria ochroleuca* tel que la Laminaïne^{®} de Secma ; l'essence de Mamaku de Lucas Meyer, l'extrait de lupin commercialisé par la société SILAB sous la dénomination commerciale Structurine^{®} ; l'extrait de bourgeons de hêtre *Fagus sylvatica* commercialisé par la société GATTEFOSSE sous la dénomination commerciale Gatuline^{®} RC.

### Agents stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8^{®} ou commercialisé par la société SILAB sous la dénomination commerciale Raffermine^{®}) ; un extrait de protéines hydrolysées de soja tel que le RIDULISSE^{®} de SILAB ; et les hormones végétales telles que les giberrellines et les cytokinines ; un extrait peptidique de noisette tel que celui commercialisé par la société Solabia sous la dénomination Nuteline C^{®}.

De préférence on utilisera un agent favorisant la prolifération et/ou la différenciation des kératinocytes.

Les agents stimulant la prolifération des kératinocytes, utilisables dans la composition selon l'invention, comprennent notamment le phloroglucinol, l'extrait de feuille d'hydrangea macrophylla comme l'Amacha liquid E^{®} d'Ichimaru Pharcos, un extrait de levure tel que le Stimoderm^{®} de CLR ; l'extrait de *Larrea divaricata* tel que le Capislow^{®} de Sederma, les mélanges d'extraits de papaye, de feuilles d'olivier et de citron tel que la Xyléine^{®} de Vincience, l'extrait de feuille d'hydrangea macrophylla comme l'Amacha liquid E^{®} d'Ichimaru Pharcos, le rétinol et ses esters dont le palmitate de rétinyle, les extraits de tourteaux de noix commercialisés par Gattefosse et les extraits de *solanum tuberosum* tel que le Dermolectine^{®} commercialisé par Sederma.

Parmi les agents stimulant la différenciation des kératinocytes comprennent par exemple les minéraux tels que le calcium; un extrait peptidique de lupin tel que celui commercialisé par la société SILAB sous la dénomination commerciale Structurine^{®}; le beta-sitosteryl sulfate de sodium tel que celui commercialisé par la société SEPORGA sous la dénomination commerciale Phytocohésine^{®} ; et un extrait hydrosoluble de maïs tel que celui commercialisé par la société SOLABIA sous la dénomination commerciale Phytovityl^{®} ; un extrait peptidique de *Voandzeia substerranea* tel que celui commercialisé par la société Laboratoires Sérobiologiques sous la dénomination commerciale Filladyn LS 9397^{®} ; et les lignanes tels que le sécoisolaricirésinol, le rétinol et ses esters dont le palmitate de rétinyl.

Comme agents stimulant la prolifération et/ou la différenciation des kératinocytes, on peut citer encore les oestrogènes tel que l'oestradiol et homologues ; les cytokines.

Comme actifs stimulant la prolifération des fibroblastes ou des kératinocytes et/ou la différenciation des kératinocytes préférés, on citera des protéines ou polypeptides végétaux, extraits notamment du soja (par exemple un extrait de soja commercialisé par la société LSN sous la dénomination Eleseryl SH-VEG 8^{®} ou commercialisé par la société SILAB sous la dénomination commerciale Raffermine^{®}) ; un extrait de protéines hydrolysées de soja tel que le RIDULISSE^{®} de SILAB ; un extrait peptidique de noisette tel que celui commercialisé par la société Solabia sous la dénomination Nuteline C^{®}; l'adénosine, le phloroglucinol, un extrait de levure tel que le Stimoderm^{®} de CLR ; un extrait peptidique de lupin tel que celui commercialisé par la société SILAB sous la dénomination commerciale Structurine^{®}; un extrait hydrosoluble de maïs tel que celui commercialisé par la société SOLABIA sous la dénomination commerciale Phytovityl^{®} ; un extrait peptidique de *Voandzeia substerranea* tel que celui commercialisé par la société Laboratoires Sérobiologiques sous la dénomination commerciale Filladyn LS 9397^{®} ; le rétinol et ses esters dont le palmitate de rétinyle.

### Agents favorisant la maturation de l'enveloppe cornée

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance^{®} de National Starch et les autres actifs mentionnés dans la demande L'OREAL FR2877220.

### Inhibiteurs de NO-synthases

L'agent ayant une action d'inhibiteur de NO synthase peut être choisi parmi les OPC (oligomères procyanidoliques) ; les extraits de végétal de l'espèce *Vitis vinifera* notamment commercialisés par la société Euromed sous la dénomination Leucocyanidines de raisins extra, ou encore par la société Indena sous la dénomination Leucoselect^{®}, ou enfin par la société Hansen sous la dénomination Extrait de marc de raisin ; les extraits de végétal de l'espèce *Olea europaea* de préférence obtenus à partir de feuilles d'olivier et notamment commercialisés par la société VINYALS sous forme d'extrait sec, ou par la société Biologia & Technologia sous la dénomination commerciale Eurol^{®} BT ; les extraits d'un végétal de l'espèce *Gingko biloba* de préférence un extrait aqueux sec de ce végétal vendu par la société Beaufour sous le nom commercial Ginkgo biloba extrait standard et leurs mélanges.

### Agents stimulant le métabolisme énergétique des cellules

L'actif stimulant le métabolisme énergétique des cellules peut par exemple être choisi parmi la biotine, un extrait de *Saccharomyces cerevisiae* tel que le Phosphovital^{®} de Sederma, le mélange de sels de sodium, de manganèse, de zinc et de magnésium d'acide pyrrolidone carboxylique comme le Physiogenyl^{®} de Solabia, un mélange de gluconate de zinc, de cuivre et de magnésium tel que le Sepitonic M3^{®} de Seppic et leurs mélanges ; et un beta-glucan issu de *Saccharomyces cerevisiae,* tel que celui commercialisé par la société Mibelle AG Biochemistry.

L'invention porte également sur un procédé de traitement cosmétique de la peau destiné à diminuer ou prévenir les signes du vieillissement de la peau ou de ses phanères (cheveux, cils, ongles, ...), comprenant au moins une étape consistant à appliquer sur la peau au moins une composition telle que définie précédemment.

Le procédé selon l'invention comprend plus spécifiquement au moins une étape consistant à appliquer sur la peau de personnes présentant une peau présentant au moins l'un des signes de vieillissement cutané rappelés précédemment au moins une composition telle que définie précédemment.

Plus particulièrement, il comprend au moins une étape consistant à appliquer sur la peau de personnes présentant une peau ou une zone de peau vieillie, ridée, ou molle et/ou flasque ou sur des zones du corps présentant une perte d'élasticité et/ou de fermeté et/ou de tonicité au moins une composition telle que définie précédemment.

La composition selon l'invention peut être appliquée sur la partie de la peau ou des phanères à traiter, en particulier sur le visage, le corps, le cou, les mains, les cheveux ou le cuir chevelu, de préférence de façon quotidienne ou pluriquotidienne. L'application pourra être par exemple renouvelée tous les jours pendant une période variable selon les effets souhaités, généralement de 3 à 6 semaines, mais pourra être prolongée ou poursuivie en continu.

Selon une alternative, la composition selon l'invention peut être administrée par voie injectable en association ou non avec des produits de comblement. En effet, l'une des solutions retenues pour lutter contre les rides et/ou la perte de volume des tissus mous est l'utilisation de produits de comblement (ou « filler »). Ce comblement peut être réalisé par l'utilisation de produits non résorbables, tels que des gels de polyacrylamide ou des particules de polyméthylméthacrylate (PMMA). Néanmoins, ces composés peuvent entrainer des réactions d'intolérance du type inflammation ou hypersensibilité.

On privilégie l'utilisation de composants résorbables, tels que les protéines, les graisses, le collagène ou l'acide hyaluronique. Mais ces composés sont dégradés assez rapidement dans l'organisme, ce qui réduit leur efficacité. Pour y remédier il faut donc procéder à une réticulation plus ou moins poussée de ces composants. A ce jour, l'acide hyaluronique utilisé dans des formes pharmaceutiques ou des dispositifs médicaux se présente sous la forme d'un gel de hyaluronate de sodium. Le monosaccharide selon l'invention ou les compositions les contenant pourront également être appliqués par mésothérapie. La mésothérapie est une technique de traitement par injection intraépidermique et/ou intradermique et/ou sous-cutanée de produit(s) actif(s), comme par exemple des micro-nutriments, des vitamines, et/ou de l'acide hyaluronique. Les compositions sont administrées selon cette technique par injection sous forme de multiples gouttelettes de faible taille au niveau de l'épiderme, de la jonction dermo-épidermique et/ou du derme afin, notamment, de réaliser un nappage sous-cutané. La technique de mésothérapie est notamment décrite dans l'ouvrage « Traité de mésothérapie » de Jacques LE COZ, édition Masson, 2004. La mésothérapie faite sur le visage est également appelée mésolift, ou également sous le terme anglosaxon de « mesoglow ».

Ainsi, un autre objet de la présente invention peut être un dispositif, en particulier un dispositif médical, comprenant une quantité efficace d'au moins un monosaccharide tel que défini précédemment en association avec une quantité efficace d'au moins un agent desquamant. Ce dispositif peut être adapté à une injection intraépidermique et/ou intradermique et/ou sous-cutanée. L'association d'actifs telle que définie ci-dessus est mise en solution dans un milieu stérile. Ledit dispositif peut comprendre au moins un autre composé, comme au moins un produit résorbable ou non, tel que ceux mentionnés ci-dessus, éventuellement réticulé.

Ledit dispositif peut être par exemple une seringue avec une aiguille ou encore un dispositif injecteur sans aiguille, tel que ceux utilisés dans la technique de soin connue sous le nom de mésothérapie. Un kit comprenant un dispositif peut être également envisagé, ledit kit comprenant un dispositif, en particulier une seringue ou un dispositif injecteur, et au moins l'association d'actifs, monosaccharide(s) et agent desquamant, tel que défini ci-dessus. Ledit kit peut comprendre également une aiguille. Ledit dispositif peut se trouver prêt à l'emploi, c'est à dire pré-rempli, ou doit être rempli lors de l'utilisation. Dans ce dernier cas, une composition ou un autre dispositif (comme une ampoule) comprend ladite l'association d'actifs, monosaccharide(s) et agent desquamant, éventuellement en association avec au moins un autre composé actif, comme au moins un produit résorbable ou non, tel que les produits de comblement mentionnés ci-dessus, éventuellement réticulé.

L'injection de l'association selon l'invention peut être réalisée simultanément à, ou avant ou après, l'application sur la peau ou ses phanères d'une autre composition cosmétique ou pharmaceutique, de préférence dermatologique, comprenant, dans un support physiologiquement acceptable, au moins un autre actif, tel que cité ci-dessus.

Selon un autre aspect, l'invention concerne également un ensemble cosmétique comprenant : i) un récipient délimitant au moins un compartiment, ledit récipient étant fermé par un élément de fermeture ; et ii) une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier. L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur. L'applicateur peut être sous forme d'un pinceau, tel que décrit par exemple dans le brevet FR 2 722 380. Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage.

Alternativement, le couplage entre l'élément de fermeture et le récipient se fait autrement que par vissage, notamment via un mécanisme à baïonnette, par encliquetage, serrage, soudage, collage, ou par attraction magnétique. Par « encliquetage » on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique, comme le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube. Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Le contenu des brevets ou demandes de brevets cités précédemment sont incorporés par référence dans la présente demande.

Selon un mode particulier, l'invention concerne un ensemble cosmétique comprenant :
- une composition A contenant au moins un agent desquamant,
- une composition B, conditionnée de manière séparée de la composition A, comprenant au moins un monosaccharide choisi parmi le mannose, le rhamnose et leur mélange.

L'invention concerne enfin un procédé de traitement cosmétique ou dermatologique comprenant au moins une étape d'administration, en particulier d'application topique, sur la peau et/ou ses phanères, de la composition A et au moins une étape d'administration, en particulier d'application topique sur la peau et/ou ses phanères, de la composition B.

L'administration de la composition A selon l'invention peut être réalisée simultanément à, ou avant ou après, l'administration de la composition B. Comme spécifié précédemment, l'administration de la composition A et de la composition B peut être réalisée par voie topique, orale ou par injection.

Selon une alternative, on administre en premier lieu la composition A et en second lieu la composition B. Selon une autre alternative, on administre en premier lieu la composition B et en second lieu la composition A.

Les compositions A et B peuvent être conditionnées séparément à l'intérieur de deux compartiments, formés soit par deux récipients distincts, soit à l'intérieur d'un dispositif unitaire. Par « dispositif unitaire », on entend un dispositif par lequel les deux compartiments sont solidaires l'un de l'autre. Un tel dispositif peut être obtenu par un procédé de moulage en une seule pièce des deux compartiments, notamment en un matériau thermoplastique. Il peut également résulter de toute forme d'assemblage, notamment par collage, soudage, ou autre encliquetage.

Selon un premier mode de réalisation, les deux récipients sont indépendants l'un de l'autre. De tels récipients peuvent se présenter sous diverses formes. Il peut s'agir notamment de tubes, de flacons ou de bidons.

L'un et/ou l'autre des récipients peuvent être surmontés d'une pompe à actionnement manuel surmontée d'un bouton poussoir pour l'actionnement de la pompe et la distribution de la composition via au moins un orifice de distribution.

Alternativement, l'un et/ou l'autre des récipients sont pressurisés, notamment au moyen d'un agent propulseur, en particulier un gaz propulseur. Dans ce cas, le (ou les) récipient(s) est (sont) équipé(s) d'une valve surmontée par un bouton poussoir équipée d'une buse ou de tout autre moyen de diffusion pour la distribution du produit.

Le propulseur peut être en mélange avec la composition à distribuer ou séparé, notamment via un piston apte à coulisser à l'intérieur du récipient, ou via les parois souples d'une poche à l'intérieur de laquelle est disposée la composition.

Les récipients peuvent être constitués de matériaux divers : plastique, verre, ou métal.

Alternativement encore, les deux compartiments sont formés de deux compartiments concentriques formés à l'intérieur d'un tube, et sont surmontés d'une pompe sans reprise d'air équipée d'un bouton poussoir à un ou deux orifices de distribution. A l'intérieur du tube est prévu un piston qui remonte en direction de la pompe au fur et à mesure que les compositions sont prélevées à l'intérieur des récipients. De tels modes de distribution sont utilisés notamment pour la distribution de pâtes dentifrices.

### Légendes des Figures

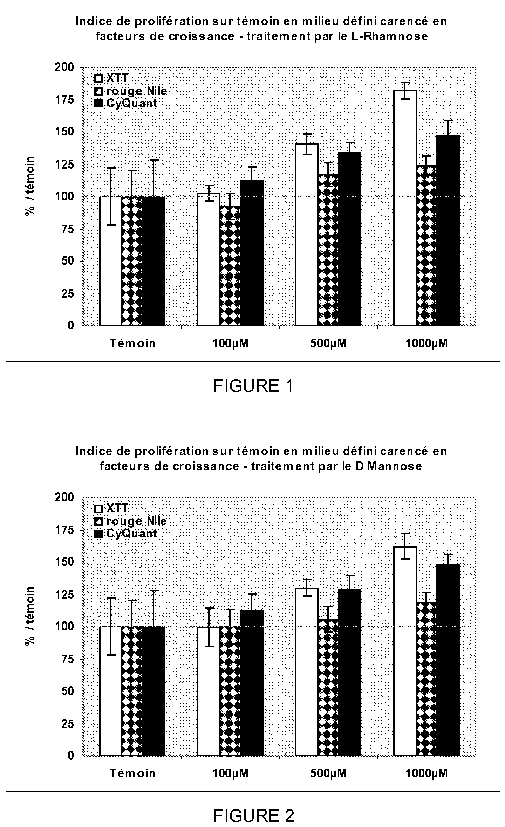

**Figure 1** **:** Diagramme schématisant les résultats obtenus pour la prolifération des kératinocytes, en présence d'un témoin, en présence de différents marqueurs, en milieu carencé en facteurs de croissance, et avec ajout de différentes concentrations en L-Rhamnose reportées en abscisse. Les valeurs reportées en ordonnée correspondent aux pourcentages de cellules marquées mesurés par rapport au témoin.
**Figure 2** **:** Diagramme schématisant les résultats obtenus pour la prolifération des kératinocytes, en présence d'un témoin, en présence de différents marqueurs, en milieu carencé en facteurs de croissance, et avec ajout de différentes concentrations en D-Mannose reportées en abscisse. Les valeurs reportées en ordonnée correspondent aux pourcentages de cellules marquées mesurés par rapport au témoin.

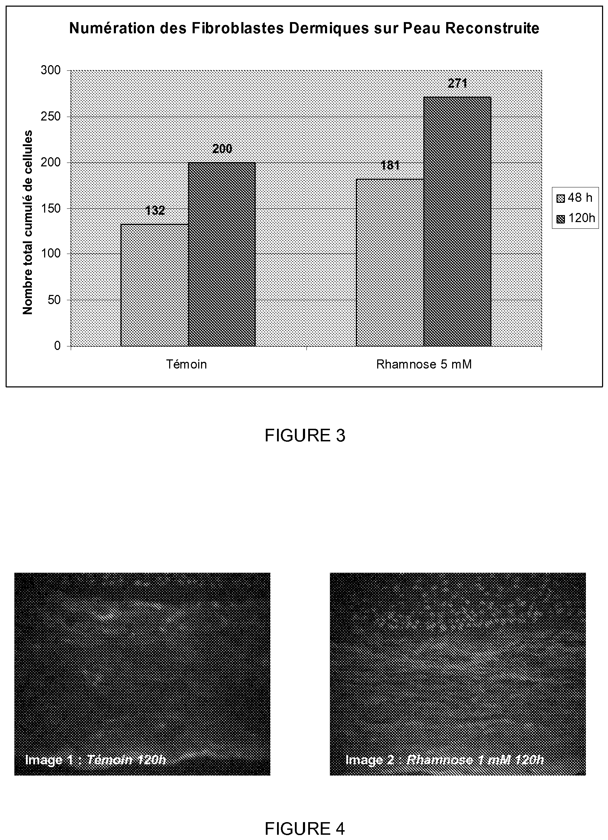

**Figure 3** **:** Diagramme représentant le nombre de fibroblastes mesurés entre une peau reconstruite complète témoin non traité, à gauche, et une peau reconstruite complète traitée par 5 mM de rhamnose, à droite. Les fibroblastes sont comptés à différents stades du traitement. Ainsi, pour chaque type de peau la colonne de gauche correspond à la numérotation effectuée à 48 h et la colonne de droite correspond à la numérotation effectuée à 120 h de traitement.
**Figure 4** **:** Photographies de coupe à congélation de peau reconstruite de 7µm d'épaisseur. Le niveau de fluorescence se matérialise par les taches blanches du cliché en noir et blanc, il est proportionnel à la quantité de procollagène de type I. A gauche figure la peau témoin et à droite la peau traitée par 1 mM de rhamnose.

L'invention est illustrée plus en détail dans les exemples suivants qui sont présentés à titre illustratif et non limitatif du domaine de l'invention.

### Exemples

### Exemple 1 : Prolifération des kératinocytes

### Protocole

Les kératinocytes (lignée HaCat) sont cultivés dans deux conditions : milieu de culture défini complet (condition standard) et milieu de culture carencé en facteurs de croissance. Ce milieu carencé entraîne un retard maitrisé de la prolifération cellulaire. Dans ces conditions, il est alors possible de mesurer les effets de composés, capables de compenser la carence en facteurs de croissance du milieu de culture et donc de relancer la multiplication cellulaire et /ou de stimuler leur métabolisme.

La prolifération kératinocytaire est mesurée au moyen de trois marqueurs sur la même population cellulaire : le taux d'ADN qui est proportionnel au nombre de cellules (sonde Cyquant), le taux de lipides polaires constitutifs de membranes cellulaires (sonde Rouge Nil) et la respiration mitochondriale, qui réflète le métabolisme cellulaire général (sonde XTT).

### Résultats

Les résultats sont donnés dans les figures 1 et 2.

Les deux monosaccharides Rhamnose et Mannose démontrent leur capacité à activer la prolifération des kératinocytes, lorsque ceux-ci sont cultivés en milieu appauvri en facteurs de croissance, condition de culture qui retarde significativement leur croissance cellulaire.

Cette activation de la prolifération cellulaire par les deux composés se manifeste par un nombre de cellules plus important par rapport au témoin non traité.

Ce nombre augmenté de cellules se matérialise par un taux d'ADN (Cyquant), un taux de lipides polaires (signal Rouge Nil) et une respiration mitochondriale (signal XTT) significativement augmentés lorsque les monosaccharides sont évalués à 1 mM. A 500 µM, les deux molécules présentent déjà une efficacité.

Les deux monosaccharides mannose et rhamnose exercent donc une influence sur la prolifération des kératinocytes. Ils activent la prolifération des kératinocytes cultivés en milieu appauvri en facteur de croissance, ce qui se manifeste par un nombre de cellules plus important par rapport au témoin non traité.

Le rhamnose et le mannose présentent donc une efficacité anti âge intéressante en venant booster le renouvellement épidermique et lutter contrer l'atrophie épidermique liée au vieillissement.

### Exemple 2 : Prolifération des fibroblastes

### Protocole

Le rhamnose a été étudié sur un modèle de peau reconstruite complète afin de mesurer son efficacité anti âge au niveau du compartiment dermique. Brièvement, le modèle de peau reconstruite utilisé est celui décrit par Bell et al, (Bell E. et al, The reconstitution of living skin, J Invest Dermatol, 1983, Jul ;81*)* : il comporte un équivalent dermique sur lequel est reconstruit un épiderme pluristratifié ; l'équivalent dermique est fabriqué à partir de collagène acido soluble, de milieu de culture contenant du sérum et de fibroblastes humains normaux adultes. Après 5 jours de rétraction, cet équivalent est ensemencé avec des kératinocytes puis cultivé durant 6 jours en immersion et 7 jours à émersion afin d'obtenir un épiderme pluristratifié et différencié présentant une couche cornée.

La peau reconstruite est traitée par du rhamnose à 5 mM pendant 2 jours et 5 jours dans le milieu de culture ; à l'issue du traitement, les peaux reconstruites sont incluses en Tissue Tek en vue de coupes à congélation de 7µM d'épaisseur au cryostat. Les coupes réalisées sont ensuite marquées à l'iodure de propidium pour marquer l'ADN des noyaux des fibroblastes en vue de leur numération. 3 coupes à congélation sont réalisées aléatoirement sur chaque peau reconstruite ; sur chaque coupe, 2 champs microscopiques (objectif X25) sont analysés en microscopie à fluorescence et photographiés. La numération des fibroblastes dermiques est donc réalisée pour chaque peau reconstruite sur 6 images au total représentant les 6 champs microscopiques considérés. Le nombre de fibroblastes dermiques est comparé entre la peau témoin et celle traitée par le rhamnose aux deux temps de la cinétique.

### Résultats

Les résultats sont donnés à la figure 3.

Il a été constaté que le rhamnose induit la stimulation de la croissance des fibroblastes dermiques de la peau reconstruite dès 48 heures de traitement, stimulation confirmée à 120 h de traitement, avec entre 30 à 35% de cellules en plus (voir figure 3). A noter que cette stimulation s'accompagne d'une stimulation de la synthèse de procollagène 1 à 5 mM, ainsi qu'à 1 mM, ce qui peut également résulter du nombre accru des fibroblastes responsable de la sécrétion de cette protéine majeure de la matrice extracellulaire.

Ces deux effets complètent l'activité anti-âge du rhamnose déjà mesurée sur le compartiment épidermique, en venant stimuler la prolifération et le métabolisme du fibroblaste, cellule majeure du compartiment dermique.

### Exemple 3 : Synthèse de Procollagène 1

Il a été procédé également sur d'autres séries de coupes à congélation à la détection classique par immunofluorescence indirecte du procollagène de type I au niveau du derme de la peau reconstruite (Anticorps anti procoll 1 *(MAB 1912 Millipore)* + conjugué couplé à FITC *(112-095-068 Jackson Immunoresearch)).* Afin de bien se repérer au sein de l'architecture cutanée lors de l'examen microscopique des coupes, les noyaux cellulaires des kératinocytes et des fibroblastes sont localisés grâce à leur marquage à l'iodure de propidium, comme décrit plus haut. 3 coupes à congélation sont réalisées aléatoirement sur chaque peau reconstruite et sur chaque coupe, 2 champs microscopiques (objectif X25) sont analysés en microscopie à fluorescence et photographiés. Les niveaux de fluorescence proportionnels à la quantité de procollagène de type I sont comparés entre la peau témoin et la peau traitée par le rhamnose.

Sur l'image 1, figure 4, correspondant à une coupe de la peau reconstruite témoin à 120h de culture, la présence du procollagène de type 1 synthétisé par les fibroblastes dermiques, se matérialise par la fluorescence verte située dans la partie inférieure de l'image. On devine sur la partie supérieure de l'image, la partie basale de l'épiderme, tissu très cellulaire, visualisable par les nombreux noyaux des kératinocytes. On visualise également dans le derme ; tissu beaucoup moins cellulaire, la distribution aléatoire des fibroblastes au sein de la matrice extracellulaire dermique. Sur l'image 2, figure 4, correspondant par exemple, à une coupe de la peau reconstruite traitée par le Rhamnose à 1 mM pendant 120 heures, on constate une nette augmentation de la fluorescence verte comparativement à celle observée pour la peau témoin (image 1) ainsi qu'une distribution du signal fluorescent matérialisant bien l'aspect fibrillaire du procollagène de type I néosynthétisé. Cette augmentation de la fluorescence générale indique que le traitement par le rhamnose a fortement stimulé la synthèse du procollagène de type I par les fibroblastes.

Ces résultats montrent bien la capacité du rhamnose à stimuler le métabolisme du fibroblaste, métabolisme qui au cours du vieillissement, se déséquilibre plus vers la dégradation de la matrice extracellulaire que vers son renouvellement.

Le rhamnose en stimulant à la fois le métabolisme et la croissance des fibroblastes dermiques démontre bien son efficacité anti-âge sur le derme, efficacité complémentaire de celle mesurée vis-à-vis du compartiment épidermique.

### Exemple 4 : Association Rhamnose et agent desquamant, Mise en évidence de la complémentarité d'action anti âge du rhamnose et d'un agent desquamant (dérivé d'acide jasmonique)

L'association rhamnose/dérivé d'acide jasmonique a été étudiée sur un modèle de peau reconstruite complète afin de mesurer son efficacité anti âge au niveau du compartiment épidermique.

Brièvement, le modèle de peau reconstruite utilisé est celui décrit par Bell et al, (Bell E. et al, The reconstitution of living skin, J Invest Dermatol, 1983, Jul ;81*)* : il comporte un équivalent dermique sur lequel est reconstruit un épiderme pluristratifié ; l'équivalent dermique est fabriqué à partir de collagène acido soluble, de milieu de culture contenant du sérum et de fibroblastes humains normaux adultes. Après 5 jours de rétraction, cet équivalent est ensemencé avec des kératinocytes puis cultivé durant 6 jours en immersion et 7 jours à émersion afin d'obtenir un épiderme pluristratifié et différencié présentant une couche cornée.

La peau reconstruite est traitée par exemple par le dérivé d'acide jasmonique à 10 µM et le rhamnose à 1 mM pendant 2 jours et 5 jours dans le milieu de culture ; à l'issue du traitement, les peaux reconstruites sont incluses en Tissue Tek en vue de coupes à congélation de 7µm d'épaisseur au cryostat. Les coupes réalisées sont ensuite marquées à l'iodure de propidium pour mar45-106Nmalpighiens en vue de leur numération. 3 coupes à congélation sont réalisées aléatoirement sur chaque peau reconstruite; sur chaque coupe, 2 champs microscopiques (objectif X25) sont analysés en microscopie à fluorescence et photographiés. La numération des kératinocytes est donc réalisée pour chaque peau reconstruite sur 6 images au total représentant les 6 champs microscopiques considérés. Le nombre de kératinocytes est comparé entre la peau témoin et celle traitée par l'association dérivé d'acide jasmonique / rhamnose aux deux temps de la cinétique.

### Résultats

L'association Rhamnose / agent desquamant (dérivé d'acide jasmonique) augmente le nombre de kératinocytes dans l'épiderme malpighien, témoignant de la double activité du rhamnose vis-à-vis de la prolifération et du dérivé d'acide jasmonique sur le Stratum Corneum.

### Exemple 5 : exemple de réalisation d'une composition cosmétique selon l'invention

| **Crèmes ANTI Age Totale : émulsion huile dans eau** | |
|---|---|
| **Ammonium Polyacryldimethyltauramide (Hostacerin AMPS de Clariant)** | **1.00%** |
| **Cyclohexasiloxane** | **5.0%** |
| **Huile d'amande d'abricot** | **7%** |
| **Isononyl isononanoate** | **7%** |
| **Stearyl alcohol** | **0.30%** |
| **Glyceryl stearate / PEG-100 stearate** | **0.70%** |
| **Dimyristyl tartrate / cetearyl alcohol / C12-15 pareth-7 / PPG-25 laureth-25** | **0.50%** |
| **Gomme de Xanthan** | **0,20%** |
| **rhamnose** | **5%** |
| **acide (1R,2R) 3-hydroxy-2-pentyl-cyclopentaneacétique** | **2%** |
| **Conservateurs** | **0.3%** |
| **Eau** | **qsp 100** |

Appliquée bi-quotidiennement pendant 6 mois, une amélioration globale de l'âge apparent du visage, via en particulier une réduction de l'apparence des rides d'expression et une amélioration de l'éclat du teint, est observée.

### Exemple 6 : exemple de réalisation d'une composition cosmétique selon l'invention

| **Crèmes Anti Age : émulsion huile dans eau** | |
|---|---|
| **Ammonium Polyacryldimethyltauramide (Hostacerin AMPS de Clariant)** | **1.00%** |
| **Cyclohexasiloxane** | **5.0%** |
| **Huile d'amande d'abricot** | **7%** |
| **Isononyl isononanoate** | **7%** |
| **Stearyl alcohol** | **0.30%** |
| **Glyceryl stearate / PEG-100 stearate** | **0.70%** |
| **Dimyristyl tartrate / cetearyl alcohol / C12-15 pareth-7 / PPG-25 laureth-25** | **0.50%** |
| **Gomme de Xanthan** | **0,20%** |
| **mannose** | **5%** |
| **Acide N-octanoyl salicylique** | **0.5%** |
| **Conservateurs** | **0.50%** |
| **Eau** | **qsp 100** |

### Exemple 7 : exemple de réalisation d'une composition cosmétique selon l'invention

| **Crèmes Anti Age : émulsion huile dans eau** | |
|---|---|
| **Ammonium Polyacryldimethyltauramide (Hostacerin AMPS de Clariant)** | **1.00%** |
| **Cyclohexasiloxane** | **5.0%** |
| **Huile d'amande d'abricot** | **7%** |
| **Isononyl isononanoate** | **7%** |
| **Stearyl alcohol** | **0.30%** |
| **Glyceryl stearate / PEG-100 stearate** | **0.70%** |
| **Dimyristyl tartrate / cetearyl alcohol / C12-15 pareth-7 / PPG-25 laureth-25** | **0.50%** |
| **Gomme de Xanthan** | **0,20%** |
| **Mannose** | **2,5%** |
| **Rhamnose** | **2,5%** |
| **Acide N-octanoyl salicylique** | **0.5%** |
| **Conservateurs** | **0.50%** |
| **Eau** | **qsp 100** |

### Exemple 8 : exemple de réalisation d'une composition cosmétique selon l'invention

### Crème de jour anti-âge pour le visage

### Phase A1 :-

- Distéarate de sucrose commercialisé par la Société STEARINERIE DUBOIS 1,75 %
- Stéarate de sorbitane oxyéthyléné à 4 moles d'oxyde d'éthylènecommercialisé par la Société ICI sous le nom "TWEEN 61" 1,15 %
- Acide stéarique 0,75 %
- Stearyl heptanoate 4,00 %
- Vaseline codex 1,50 %
- Huile d'avocat 3,20 %
- Huile de jojoba 3,00 %
- Huile de silicone volatile 2,70 %
- Acétate de vitamine E 1,00 %
- Glycérides de Vitamine F 3,00 %

### Phase A2 :

Gomme de silicone commercialisée par DOW CORNING sous le nom "Q2-1403 Fluid"3,00 %
- Propylparaben 0,2 %
- Parfum 0,3 %

### Phase B :

- Glycérine 3,00 %
- Hydroxyproline 1,00 %
- D-panthenol 1,00 %
- Triéthanolamine 0,35 %
- Rhamnose 3,00%
- acide (1R,2R) 3-hydroxy-2-pentyl-cyclopentaneacétique 2%
- Méthylparaben 0,3 %
- Eau déminéralisée q.s.p. 100 %

### Phase C :

- Ammonium Polyacryldimethyltauramide (Hostacerin AMPS de Clariant) 1%

## Revendications

1. Utilisation cosmétique d'une composition comprenant, dans un milieu physiologiquement acceptable, l'association d'au moins un monosaccharide choisi parmi le mannose, le rhamnose et leur mélange, et d'au moins un agent desquamant, pour diminuer et/ou prévenir les caractéristiques des rides et/ou ridules ou pour améliorer la densité de la peau et/ou sa fermeté, dans laquelle l'agent desquamant est choisi parmi l'acide jasmonique, l'acide gentisique, un de leurs dérivés, et les dérivés de l'acide salicylique;
le monosaccharide étant sous forme de monomère;
les dérivés d'acide salicylique étant choisis parmi l'acide n-octanoyl-5-salicylique (ou acide capryloyl salicylique) ; l'acide n-décanoyl-5-salicylique ; l'acide n-dodécanoyl-5-salicylique ; l'acide n-heptyloxy-5-salicylique et leurs sels correspondants; et
l'acide jasmonique et ses dérivés étant choisis parmi :
- l'acide 3-hydroxy-2-[(2Z)-2-pentenyl]- cyclopentaneacétique,
- le 3-hydroxy-2-[(2Z)-2-pentenyl]- cyclopentaneacétate de méthyle,
- le 2-[(2Z)-2-pentenyl]-3-hydroxy-cyclopentaneéthanol,
- l'acide 3-hydroxy-2-pentyl-cyclopentaneacétique,
- le 3-hydroxy-2-pentyl-cyclopentaneacétate de méthyle,
- le 2-pentyl-3-hydroxy-cyclopentaneéthanol, et
- l'acide jasmonique.

2. Utilisation selon la revendication 1, pour traiter, de manière préventive ou curative, les rides et/ou ridules, la peau flétrie, le manque d'élasticité et/ou de tonus de la peau, l'amincissement du derme, la dégradation des fibres de collagène, la peau molle et/ou la peau amincie.

3. Utilisation selon l'une quelconque des revendications 1 et 2, dans laquelle le monosaccharide est le mannose.

4. Utilisation selon l'une quelconque des revendications 1 à 3, dans laquelle le monosaccharide est le rhamnose.

5. Utilisation selon la revendication 4, dans laquelle le dérivé d'acide salicylique est l'acide n-octanoyl-5-salicylique.

6. Utilisation selon l'une quelconque des revendications 1 à 5, dans laquelle le dérivé d'acide jasmonique est l'acide 3-hydroxy-2-pentyl-cyclopentaneacétique.

## Patentansprüche

1. Kosmetische Verwendung einer Zusammensetzung, umfassend in einem physiologisch verträglichen Medium die Kombination aus wenigstens einem Monosaccharid, ausgewählt aus Mannose, Rhamnose und einem Gemisch davon, und wenigstens einem Mittel zum Entfernen von Hautschuppen, zur Verringerung und/oder Prävention der Charakteristika von Falten und/oder Fältchen oder zur Verbesserung der Dichte und/oder der Festigkeit der Haut, wobei das Mittel zum Entfernen von Hautschuppen aus Jasmonsäure, Gentisinsäure, einem ihrer Derivate und Salicylsäure-Derivaten ausgewählt ist;
wobei das Monosaccharid als Monomer vorliegt;
wobei die Salicylsäure-Derivate aus n-Octanoyl-5-salicylsäure (oder Capryloylsalicylsäure); n-Decanoyl-5-salicylsäure; n-Dodecanoyl-5-salicylsäure; n-Heptyloxy-5-salicylsäure und ihren korrespondierenden Salzen ausgewählt sind; und
wobei die Jasmonsäure und ihre Derivate ausgewählt sind aus:
- 3-Hydroxy-2-[(2Z)-2-pentenyl]-cyclopentanessigsäure,
- 3-Hydroxy-2-[(2Z)-2-pentenyl]-cyclopentanessigsäuremethylester,
- 2-[(2Z)-2-Pentenyl]-3-hydroxy-cyclopentanethanol,
- 3-Hydroxy-2-pentyl-cyclopentanessigsäure,
- 3-Hydroxy-2-pentyl-cyclopentanessigsäuremethylester,
- 2-Pentyl-3-hydroxy-cyclopentanethanol, und
- Jasmonsäure.

2. Verwendung gemäß Anspruch 1, zur präventiven oder kurativen Behandlung von Falten und/oder Fältchen, der welken Haut, der mangelnden Elastizität und/oder Spannung der Haut, des Dünnerwerdens der Dermis, des Abbaus von Kollagenfasern, der schlaffen Haut und/oder der dünner gewordenen Haut.

3. Verwendung gemäß einem der Ansprüche 1 und 2, wobei das Monosaccharid Mannose ist.

4. Verwendung gemäß einem der Ansprüche 1 bis 3, wobei das Monosaccharid Rhamnose ist.

5. Verwendung gemäß Anspruch 4, wobei das Salicylsäure-Derivat n-Octanoyl-5-salicylsäure ist.

6. Verwendung gemäß einem der Ansprüche 1 bis 5, wobei das Jasmonsäure-Derivat 3-Hydroxy-2-pentyl-cyclopentanessigsäure ist.

## Claims

1. A cosmetic use of a composition comprising, in a physiologically acceptable medium, a combination of at least one monosaccharide chosen from mannose, rhamnose and a mixture thereof, and of at least one desquamating agent, for reducing and/or preventing the characteristics of wrinkles and/or fine lines, or for improving the density and/or firmness of the skin, wherein the desquamating agent is chosen from jasmonic acid, gentisic acid, a derivative thereof, and salicylic acid derivatives;
the monosaccharide being a monomer;
the salicylic acid derivatives being chosen from 5-n-octanoylsalicylic acid (or capryloylsalicylic acid); 5-n-decanoylsalicylic acid; 5-n-dodecanoylsalicylic acid; 5-n-heptyloxysalicylic acid, and the corresponding salts thereof; and
the jasmonic acid and derivatives thereof being chosen from:
- 3-hydroxy-2-[(2Z)-pentenyl]cyclopentaneacetic acid,
- methyl 3-hydroxy-2-[(2Z)-pentenyl]cyclopentaneacetate,
- 2-[(2Z)-2-pentenyl]-3-hydroxycyclopentaneethanol,
- 3-hydroxy-2-pentylcyclopentaneacetic acid,
- methyl 3-hydroxy-2-pentylcyclopentaneacetate,
- 2-pentyl-3-hydroxycyclopentaneethanol, and
- jasmonic acid.

2. The use according to Claim 1, for preventively or curatively treating wrinkles and/or fine lines, withered skin, lack of skin elasticity and/or tonicity, thinning of the dermis, degradation of collagen fibres, flaccid skin and/or thinned skin.

3. The use according to any one of claims 1 and 2, wherein the monosaccharide is mannose.

4. The use according to any one of claims 1 and 2, wherein the monosaccharide is rhamnose.

5. The use according to claim 4, wherein the salicylic acid derivative is 5-n-octanoylsalicylic acid.

6. The use according to any one of claims 1 to 5, wherein the jasmonic acid, derivative is 3-hydroxy-2-pentylcyclopentaneacetic acid.
